# EUROPEAN PATENT SPECIFICATION

(11) **EP 2 997 144 B1**
(45) Date of publication and mention of the grant of the patent: **04.09.2019**
(21) Application number: 14726092.1
(22) Date of filing: 07.05.2014
(51) Int. Cl.: C12N 9/24, C12P 3/00, C12P 7/08, C12P 7/40, C12P 7/46, C12P 7/52, C12P 7/54, C12P 7/56, C12P 7/64, C12N 9/42

(54) **ENZYME COMPOSITIONS FOR THE IMPROVEMENT OF FERMENTATION PROCESSES AND BY-PRODUCTS**
ENZYMZUSAMMENSETZUNGEN ZUR VERBESSERUNG VON FERMENTATIONSVERFAHREN UND NEBENPRODUKTEN
COMPOSITIONS D'ENZYMES POUR L'AMÉLIORATION DE PROCÉDÉS DE FERMENTATION ET DE SOUS-PRODUITS

(30) Priority: 18.05.2013 EP 13168397
(43) Date of publication of application: 23.03.2016
(73) Proprietor: Direvo Industrial Biotechnology GmbH, 50829 Köln (DE); BASF Enzymes, LLC, San Diego, CA 92121 (US)
(72) Inventor: MILOS, Klaudija, 50829 Köln (DE)
(74) Representative: Roth, Andy Stefan
(86) International application number: PCT/EP2014/059293
(87) International publication number: WO 2014/187668

(56) References cited:
- WO-A1-2012/084225
- WO-A2-2007/095398
- WO-A2-2008/008070
- WO-A2-2009/018537
- WO-A2-2010/059424
- WO-A2-2010/066411

## Description

### FIELD OF THE DISCLOSURE

The present disclosure relates to novel enzyme compositions for the use in processes of producing a fermentation product, in particular ethanol. The use of the disclosed enzyme composition results in improving the quality of by-products in the fermentative production process since they are capable of optimal degrading components in the fermented mash in the fermentation process.

### BACKGROUND OF THE DISCLOSURE

Fermentation products, such as ethanol, are produced by first degrading starch- containing material into fermentable sugars by liquefaction and saccharification and then converting the sugars directly or indirectly into the desired fermentation product using a fermenting organism. Liquid fermentation products such as ethanol are recovered from the fermented mash (often referred to as "beer" or "beer mash"), e.g., by distillation, which separate the desired fermentation product from other liquids and/or solids. The remaining faction, referred to as "whole stillage", is dewatered and separated into a solid and a liquid phase, e.g., by centrifugation. The solid phase is referred to as "wet cake" (or "wet grains" or "WDG") and the liquid phase (supernatant) is referred to as "thin stillage". Dewatered wet cake is dried to provide "Distillers Dried Grains" (DDG) used as nutrient in animal feed. Thin stillage is typically evaporated to provide condensate and syrup (or "thick stillage") or may alternatively be recycled directly to the slurry tank as "backset". Condensate may either be forwarded to a methanator before being discharged or may be recycled to the slurry tank. The syrup consisting mainly of limit dextrins and non-fermentable sugars may be blended into DDG or added to the wet cake before drying to produce DDGS (Distillers Dried Grain with Solubles).

It is known to commercially use the various byproducts and residues derived from the fermentation processes like the ethanol production process. Distillers residues or byproducts, as well as by-products of cereal and other food industry manufacturing, are known to have a certain value as sources of protein and energy for animal feed. Furthermore, the oil from the by-products like DDGS can be recovered as a separate by-product for use in biodiesel production or other biorenewable products are sought.

The by-products like DDG, DDGS or WDG comprises proteins, fibers, fat and unconverted starch. For example DDGS contains typically about 30% of protein. While the protein content is high the amino acid composition is not well suited for monogastric animals if used as animal feed. In general processing of DDGS, especially drying time and temperature are effecting the availability and digestibility of the amino acids, especially lysine.

Furthermore, the by-products are mainly fibrous by-products comprising Crude Fibers (CF), which are structural carbohydrates consisting of cellulose, hemicellulose and indigestible materials like lignin. The structural carbohydrates are not digestible in animal's small intestine. Fibers are characterized and analyzed by different methods and can be divided into crude fibers (CF), neutral detergent fibers (NDF) and acid detergent fibers (ADF). The proportion of cellulose and lignin in the crude fibers fraction also determines the digestibility of crude fibers or its solubility in the intestine. High cellulose and lignin concentrations mean reduced digestibility and vice versa. Hemicelluloses are capable to bind water. The soluble part of fibres the soluble non-starch-polysaccharides (NSP) cannot be digested by monogastric animals like swine and poultry, but increase viscosity, due to their capability to bind water, and are a nutritional constraint, since they can cause moist, sticky droppings and wet litter. The antinutritional effect of soluble NSP's is mainly related to the increase in digesta viscosity. The increased viscosity is slowing down the feed passage rate and hinders the intestinal uptake of nutrients and can lead to decreased feed uptake The viscosity increase a) hinders the intestinal absorption of nutrients and can result in negative effect on the consistency on faces and even symptoms of diarrhea, b) slowing down the feed passage rate and possibly to decreased feed intake. Another effect of NSP's is the so-called "Nutrient Encapsulation". The NSP's in plant cell wall encapsulated starch, protein, oil and other nutrients within the plant cell which is an impermeable barrier preventing full utilization of the nutrients within the cell.

Furthermore the soluble NSP's are responsible for high viscosities during fermentation and are directly influencing separation and drying conditions of fermentation by-products like DDGS in the production process. The bound or encapsulated water in the product is difficult to remove and causes the use of higher drying temperatures and also longer drying time, adversely affecting the quality of temperature-sensitive products like amio acids. The availability and digestibility of essential amino acids in the by-products are lowered by high temperatures and long drying time during production. Examples for NSPs are arabinoxylans, beta-glucans, galactomannans and alpha-galactosides.

As the by-products are used in animal feed for monogastrics animals like pigs and poultry it is important that the by-products have high concentrations of protein with a good amino acid composition and high availability and low soluble fibers content.

Therefore, the two ways for an improvement of a fermentative production plant ton increase their efficiency and profitability are an improved production process and the improvement of the quality of the by-products.

In the prior art, a lot of specific processes or treatment methods are described to improve fermentative production processes.

For example, WO 2007/056321 A1 discloses a method of dewatering whole stillage comprising adding enzymes to whole stillage in the ethanol production to improve the solid-liquid separation in the process.

WO 02/38786 describes a process of ethanol production, whereby enzymes are used for thinning the liquefied whole grain mash and the thin stillage. Enzymes are applied to the liquefied mash before the fermentation starts as well as to the thin stillage after centrifugation of the whole stillage.

The US 2006/0275882 A1 describes a process for producing a fermentation product wherein the viscosity of the mash is reduced by the application of enzymes before or during the fermentation.

The US 2006/0233864 A1 describes a method for improving the nutritional quality of fibrous by-products for a food manufacturing process, wherein the fibrous by-products like DDGS are inoculated with a filamentous fungus to improve the quality of the by-product.

Some ethanol plants use milo, wheat, or barley in the fermentation process, depending on geographical location and time of the year. As a result, nutrient composition can vary among DDGS sources. Because of the near complete fermentation of starch, the remaining amino acids, fat, minerals and vitamins increase approximately three-fold in concentration compared to levels found in corn. Despite the significant increase in crude protein, the poor amino acid balance of DDGS must be addressed when formulating swine and poultry diets.

Therefore, it is an object of the present disclosure to provide improved enzyme compositions and methods for improving the quality of the by-products from fermentation processes. It is further a need for enzyme compositions and methods for further improvement of the process ability by dewatering the stillage and to provide improved methods for increasing the amount of recoverable oil.

### SUMMARY OF THE DISCLOSURE

The present disclosure relates to novel enzyme compositions and uses thereof for the improvement of the quality of the by-products or residues derived from fermented mash . The present invention is only defined by the claims. The terms "embodiments" and "aspects of the invention" etc. as listed below, are for illustrative purposes alone.

In a first aspect, present disclosure relates to enzyme compositions comprising a xylanase, a beta 1,3 glucanase and a polypeptide having cellobiohydrolase II activity comprising an amino acid sequence that is at least 80 percent identical to a CBHII from Trichoderma reesei (SEQ ID NO: 1), wherein the amino acid sequence of said polypeptide comprises at least one variation as compared with SEQ ID NO: 1, and wherein the variation occurs at position corresponding to amino acid residue 438 of SEQ ID NO: 1, and wherein said variation can comprise a substitution, deletion or insertion.

In a second aspect, the present disclosure relates to methods to improve the quality of by-products or residues derived from starch-containing material in a processes for producing fermentation products comprising the steps of: i) subjecting the fermented mash during and/or after the fermentation to an enzyme composition according to any one of claims 1 to 7, ii) separating the desired fermentation product.

In a third aspect, the present disclosure relates to methods of producing a fermentation product from starch containing material, said method comprising the steps of:
i) Converting starch containing material to fermentable sugars
ii) Fermentation of the fermentable sugars with a microorganism to fermented mash
iii) Subjecting the fermented mash after the fermentation process to an enzyme composition according to the present disclosure
iv) Separation of the fermentation product

In a third aspect, the present disclosure relates to methods of recovering oil from a by-product derived from starch-containing material in a process for producing fermentation products, comprising the steps of:
i) Converting starch containing material to fermentable sugars
ii) Fermentation of the fermentable sugars in a fermentation medium into a fermentation product with a fermenting microorganism
iii) Subjecting the fermented mash after the fermentation and/or the fermentation medium during the fermentation to an enzyme composition according to the present disclosure
iv) Separation of the fermentation product
v) Separation of the by-product
vi) Recovering the oil from the by-product

In a fourth aspect, the present disclosure relates to the use of an enzyme composition according to the present disclosure for the improvement of the nutritional quality of the by-products or residues derived from fermented mash in a fermentative production process.

### BRIEF DESCRIPTION OF THE DRAWING

Figure 1 schematically shows an ethanol production process.
Figure 2 shows wildtype sequence of *Trichoderma reesei* CBHII protein. The sequence contains an N-terminal signal sequence for secretion (underlined with solid line; amino acid 1-24) followed by a Fungal-type cellulose-binding domain (= "Small four-cysteine binding domain of fungi"; underlined with broken line; amino acid 31-62).

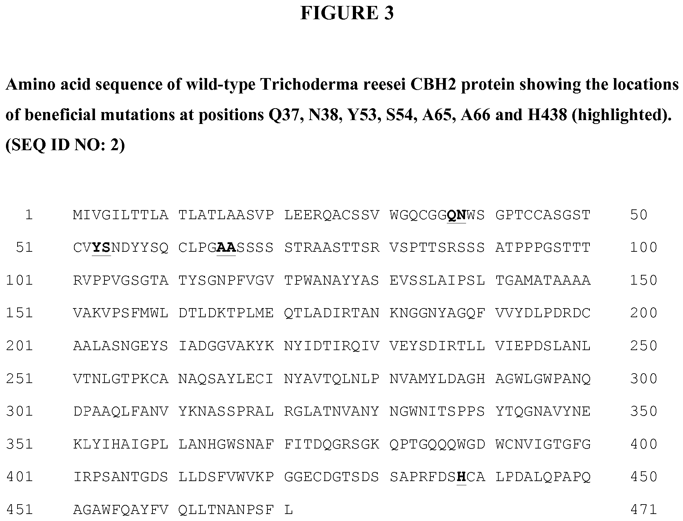

Figure 3 shows the locations of one embodiment with beneficial mutations at positions Q37, N38, Y53, S54, A65, A66 and H438 of *Trichoderma reesei* CBHII (highlighted).

### DESCRIPTION OF THE DISCLOSURE

The object of the present invention is to provide novel enzyme compositions useful for improving fermentative production processes due to a better process ability and to provide by-products from the fermentation process with an improved quality.

In advantageous embodiment, the enzyme compositions comprise a xylanase, a beta-1,3-glucanase and a CBHII. In another embodiment, the enzyme composition comprises a beta-1,3-glucanase, a 1,6-beta-glucanase, a xylanase and a CBHII.

Beta-1,3-glucanases as used herein are enzymes capable of degrading of glucan. Glucan and chitin are far more resistant to microbial degradation than cellulose, which is the major constituent of the cell wall of many yeasts and fungi-like organisms. Glucan is predominantly beta -1,3-linked with some branching via 1,6-linkage (Manners et al., Biotechnol. Bioeng, 38, p. 977, 1973), and is known to be degradable by certain beta -1,3-glucanase systems. beta -1,3-glucanase includes the group of endo-beta -1,3-glucanases also called laminarinases (E.C. 3.2.1.39 and E.C. 3.2.1.6, Enzyme Nomenclature, Academic Press, Inc. 1992).

A number of beta -1,3-glucanase genes and uses thereof have been disclosed in the prior art. An example is DD 226012 (Akad. Wissenshaft, DDR) which concerns a method for production of a Bacillus beta -1,3-glucanase. Further, JP 61040792 A (DOI K) describes a cell wall-cytolase beta -1,3-glucanase recombinant plasmid for removing the cell walls of yeast. The gene is derived from Arthrobacter and is transformed in Escherichia group bacteria. EP 440.304 concerns plants provided with improved resistance against pathogenic fungi transformed with at least one gene encoding an intracellular chitinase, or in intra- or extracellular beta -1,3-glucanase. The matching recombinant polynucleotides is also disclosed. WO 87/01388 (The Trustees of Columbia University) describes a method for preparing cell lytic enzymes, such as beta -1,3-glucanases, which can be produced by Oerksovia. WO 92/03557 (Majesty (Her) in Right of Canada) discloses a recombinant DNA expression vector comprising a 2.7 kb DNA sequence, derived from Oerskovia xanthineolytica, encoding a beta -1,3-glucanase. From WO 92/16632 a recombinant DNA sequence coding for a novel protein with beta -1,3-glucanase activity, is known.

Examples for commercial available beta -1,3-glucanase are Rohalase BX from AB Enzymes and Rapidase Glucalees from DSM.

In an embodiment the xylanase may preferably be of microbial origin, such as of fungal origin (e.g., Aspergillus, Fusarium, Humicola, Meripilus, Trichoderma) or from a bacterium (e.g., Bacillus). In a preferred embodiment the xylanase is derived from a filamentous fungus, preferably derived from a strain of Aspergillus, such as Aspergillus aculeatus; or a strain of Humicola, preferably Humicola lanuginosa. Examples of xylanases useful in the methods of the present invention include, but are not limited to, Aspergillus aculeatus xylanase (GeneSeqP:AAR63790; WO 94/21785), Aspergillus fumigatus xylanases (WO 2006/078256), and Thielavia terrestris NRRL 8126 xylanases (WO 2009/079210). The xylanase may preferably be an endo-1,4-beta-xylanase, more preferably an endo-1 ,4-beta-xylanase of GH 10 or GH 11. Examples of commercial xylanases include SHEARZYME(TM), BIOFEED WHEAT(TM), HTec and HTec2 from Novozymes A/S, Denmark.

Examples of beta-xylosidases useful in the methods of the present invention include, but are not limited to, Trichoderma reesei beta-xylosidase (UniProtKB/TrEMBL accession number Q92458), Talaromyces emersonii (SwissProt accession number Q8X212), and Neurospora crassa (SwissProt accession number Q7SOW4).

According to the invention beer may in step i) be subjected to an effective amount of any xylanase (EC 3.2.1.8), such as any of below mentioned xylanases. Xylanase activity may be derived from any suitable organism, including fungal and bacterial organisms. Fungal xylanases may be derived from strains of genera including Aspergillus, Disporotrichum, Penicillium, Neurospora, Fusarium and Trichoderma. Examples of suitable bacterial xylanases include include xylanases derived from a strain of Bacillus, such as Bacillus subtilis, such as the one disclosed in US patent no. 5,306,633.

Exo-cellobiohydrolase II (Cellobiohydrolase II, or CBH II) refers to the cellobiohydrolases which degrade cellulose by hydrolyzing the cellobiose from the reducing end of the cellulose polymer chains. The cellobiohydrolase II group belongs to the same EC group that is EC 3.2. 1.91, as the cellobiohydrolase I group, the difference being that cellobiohydrolase I degrade cellulose by hydrolyzing the cellobiose from the non-reducing end of the cellulose polymer chains.

In advantageous embodiments the enzyme compositions according to the present disclosure comprises a CBH II described in the WO 2010/066411, in particular a polypeptide having cellobiohydrolase II activity comprising an amino acid sequence that is at least 80 percent identical to a CBHII from *Trichoderma reesei* (SEQ ID NO: 1), wherein the amino acid sequence of said polypeptide comprises at least one variation as compared with SEQ ID NO: 1, and wherein the variation occurs at position corresponding to amino acid residue 438 of SEQ ID NO: 1, and wherein said variation can comprise a substitution, deletion or insertion. Regarding to the CBHII wildtype protein of Trichoderma reesei (SEQ. NO.I) Further beneficial variations, in particular substitutions can be located at seven positions (Q37, N38, Y53, S54, A65, A66 and H438) corresponding to CBHII from *Trichoderma reesei* (SEQ ID NO: 1).

Mutations or variations are described by use of the following nomenclature: position; substituted amino acid residue(s). According to this nomenclature, the substitution of, for instance, an alanine residue for a glycine residue at position 20 is indicated as 20G. When an amino acid residue at a given position is substituted with two or more alternative amino acid residues, these residues are separated by a comma or a slash. For example, substitution of alanine at position 20 with either glycine or glutamic acid is indicated as 20G/E, or 20G, 20E.

Furthermore, the following nomenclature could also be used: amino acid residue in the protein scaffold; position; substituted amino acid residue(s). According to this nomenclature, the substitution of, for instance, an alanine residue for a glycine residue at position 20 is indicated as Ala20Gly or A20G, or 20G. The deletion of alanine in the same position is shown as Ala20* or A20*. The insertion of an additional amino acid residue (e.g. a glycine) is indicated as Ala20AlaGly or A20AG. The deletion of a consecutive stretch of amino acid residues (e.g. between alanine at position 20 and glycine at position 21) is indicated as Δ(Ala20-Gly21) or Δ(A20-G21). When a sequence contains a deletion in comparison to the parent protein used for numbering, an insertion in such a position (e.g. an alanine in the deleted position 20) is indicated as *20Ala or *20A. Multiple mutations are separated by a plus sign or a slash. For example, two mutations in positions 20 and 21 substituting alanine and glutamic acid for glycine and serine, respectively, are indicated as A20G+E21S or A20G/E21S. When an amino acid residue at a given position is substituted with two or more alternative amino acid residues these residues are separated by a comma or a slash. For example, substitution of alanine at position 30 with either glycine or glutamic acid is indicated as A20G,E or A20G/E, or A20G, A20E. When a position suitable for modification is identified herein without any specific modification being suggested, it is to be understood that any amino acid residue may be substituted for the amino acid residue present in the position. Thus, for instance, when a modification of an alanine in position 20 is mentioned but not specified, it is to be understood that the alanine may be deleted or substituted for any other amino acid residue (i.e. any one of R, N, D, C, Q, E, G, H, I, L, K, M, F, P, S, T, W, Y and V).

One aspect of the present disclosure relates to methods for improving the quality of by-products or residues derived from starch-containing material in a processes for producing fermentation products comprising the steps of: i) subjecting the fermented mash during and/or preferably after the fermentation to an enzyme composition according to the present disclosure capable of degrading one or more fermented mash components, ii) separating the desired fermentation product.

By-products or residues of the fermenting process include oil, distillers' grain, brewer's grains, dried distiller's grains, dried distiller's solubles, distiller's dried grains with solubles, WDG or/and residues of the cereal processing industry, or mixtures thereof. For example, DDGS are the dried residue remaining after the starch fraction of corn is fermented with selected yeasts and enzymes to produce ethanol and carbon dioxide. After complete fermentation, the alcohol is removed by distillation and the remaining fermentation residues are dried.

Stillage is the product which remains after the mash has been converted to sugar, fermented and distilled into ethanol. Stillage can be separated into two fractions, such as, by centrifugation or screening: (1) wet cake (solid phase) and (2) the thin stillage (supernatant). The solid fraction or distillers' wet grain (DWG) can be pressed to remove excess moisture and then dried to produce distillers' dried grains (DDG). After ethanol has been removed from the liquid fraction, the remaining liquid can be evaporated to concentrate the soluble material into condensed distillers' solubles (DS) or dried and ground to create distillers' dried solubles (DDS). DDS is often mixed with DDG to form distillers' dried grain with solubles (DDGS). DDG, DDGS, and DWG are collectively referred to as distillers' grain(s).

In one embodiment of the present disclosure the enzyme composition according to the present disclosure were added during and/or preferably after the fermentation in the production process to the fermented mash and before the separation step like distillation, where the desired fermentation main product is separated from the rest of the fermented mash. The enzymes according to the present disclosure were capable of degrading components in the fermented mash (beer or beer mash) which improves the quality of by-products or residues like brewer's grains, dried distiller's grains, dried distiller's solubles, distiller's dried grains with solubles, WDG or/and residues of the cereal processing industry, or mixtures thereof. Components of the fermented mash can be cell walls, cell-walls of fermenting microorganisms, microorganisms, fibers etc.

Surprisingly, the degradation of the fermenting microorganisms itself by adding the enzyme composition according to the present disclosure, in particular by using the beta 1,3 glucanase and/or the beta 1,6 glucanase in coobination with a xylanase and the CBH II as described herein results in an increase of the nutrition content in the beer mash which results in an improvement of the nutrition quality like the protein content of the byproducts, as well as reduction of NSPs resulting from the fermentative organism cell wall like the yeast cell wall.

Therefore, in advantageous embodiments, the enzyme compositions used in the methods according to the present disclosure are capable to degrade the cell wall components of the fermenting organisms after the fermentation step as well as the fibers in the beer.

In one aspect of the present disclosure, the quality of by-products from a fermentative production process like DDG, DDGS or WDG can be improved due to the use of the enzyme composition and the methods according to the present disclosure by reducing the fiber content of the by-products.

Another aspect of the present disclosure is a better dewatering of the whole stillage from the fermentative production process to improve the drying conditions of the by-products.

Yet another aspect of the present disclosure is the improved evaporation of water from the thin stillage to improve the production and composition of the thick stillage or syrup.

In another aspect of the present disclosure, the amount of recoverable oil is increased. DDGS following an ethanol production process from corn typically contains about 13% oil, 31 % protein and 56% carbohydrates and other components. Removal of some of the oil from the DDGS will improve the quality of the DDGS for the feed market as many feed producers prefer less oil and fat in the DDGS to make high quality feed.

The method and enzyme composition of the present disclosure may be used on beer derived from production of any suitable fermentation product. The feedstock for producing the fermentation product may be any starch- and/or sugar containing material, preferably starch- and/or sugar containing plant material, including: sugar cane, tubers, roots, whole grain; and any combination thereof.

The starch-containing material may be obtained from cereals. Suitable starch-containing material includes corn (maize), wheat, barley, cassava, sorghum, rye, triticale, potato, or any combination thereof.

Corn is the preferred feedstock, especially when the fermentation product is ethanol. The starch-containing material may also consist of or comprise, e.g., a side stream from starch processing, e.g., C6 carbohydrate containing process streams that may not be suited for production of syrups. Beer components include fiber, hull, germ, oil and protein components from the starch-containing feedstock as well as non-fermented starch, yeasts, yeast cell walls and residuals. Production of a fermentation product is typically divided into the following main process stages: a) Reducing the particle size of starch-containing material, e.g., by dry or wet milling; b) Cooking the starch-containing material in aqueous slurry to gelatinize the starch, c) Liquefying the gelatinized starch-containing material in order to break down the starch (by hydrolysis) into maltodextrins (dextrins); d) Saccharifying the maltodextrins (dextrins) to produce low molecular sugars (e.g., DP1- 2) that can be metabolized by a fermenting organism; e) Fermenting the saccharified material using a suitable fermenting organism directly or indirectly converting low molecular sugars into the desired fermentation product; f) Recovering the fermentation product, e.g., by distillation in order to separate the fermentation product from the fermentation mash.

As mentioned above beer (or fermented mash) is the fermentation product consisting of ethanol, other liquids and solids of a desired fermentation product. According to the invention the fermentation product may be any fermentation product, including alcohols (e.g., ethanol, methanol, butanol, 1,3-propanediol); organic acids (e.g., citric acid, acetic acid, itaconic acid, lactic acid, gluconic acid, gluconate, succinic acid, 2,5-diketo-D-gluconic acid); ketones (e.g., acetone); amino acids (e.g., glutamic acid); gases (e.g., H₂ and CO₂), and more complex compounds, including, for example, antibiotics (e.g., penicillin and tetracycline); enzymes; vitamins (e.g., riboflavin, B12, beta-carotene); and hormones. Fermentation is also commonly used in the production of consumable alcohol (e.g., spirits, beer and wine), dairy (e.g., in the production of yogurt and cheese), leather, and tobacco industries. In a preferred embodiment the fermentation product is a liquid, preferably an alcohol, especially ethanol. The beer contemplated according to the invention may be the product resulting from a fermentation product production process including above mentioned steps a) to f). However, the beer may also be the product resulting from other fermentation product production processes based on starch- and/or lignocellulose containing starting material.

The fermenting organism may be a fungal organism, such as yeast, or bacteria. Suitable bacteria may e.g. be *Zymomonas* species, such as *Zymomonas mobilis* and *E. coli.* Examples of filamentous fungi include strains of *Penicillium* species. Preferred organisms for ethanol production are yeasts, such as e.g. *Pichia* or *Saccharomyces.* Preferred yeasts according to the disclosure are *Saccharomyces* species, in particular *Saccharomyces cerevisiae* or baker's yeast.

In a further embodiment, the solids from the fermentation step can be fractionated. After fermentation large pieces of fibers could be removed prior or after distillation. Removal can be effected with a surface skimmer before to distillation of beer. The material can be separated from the ethanol/water mix by, e.g. centrifugation. Alternatively, fibers and germs can be removed by screening the whole stillage after distillation or the grinded grains before fermentation. After germs and large pieces of fibers are removed the remaining beer or whole stillage are treated with enzymes or enzyme combinations to further improve the nutritional quality of the final byproduct like DDGS to be used.

Based on whole stillage or WDG in one embodiment of the present disclosure an additional fermenter can be introduced for an onsite enzyme production, producing a plant and process specific enzyme mixture with filamentous fungi. The supernatant of this fermentation comprising enzymes is directly transferred into the main fermenter or beer well in order to reduce the fiber content and improve quality and nutritional factors of DDGS, WDG and/or other by products.

Furthermore, the use of the enzyme compositions according to the present disclosure in the beer mash after the fermentation and before the distillation process can reduce the viscosity of the beer mash through the degradation of fibers and/or the fermentative microorganisms in the beer. The reduction of the fibers in the beer results in a reduction of the fiber content in the by-products. The early degradation of the NSP's has a direct influence on the separation and the drying conditions of the by-products like DDGS in the production process. The lower viscosity results in lower drying temperatures and also in a shorter drying time resulting in an improved quality of the by-products. For example, the temperature sensitive products like proteins and amino acids are not destroyed.

Further, by adding the enzymes according to the present disclosure to the fermented mash before the distillation step is an advantage since the enzymes in the enzyme compositions are are inactivated during the distillation.

Due to the improved quality, the by-products and residues can be used as high quality animal feed with a low fiber and oil and high protein content.

In one embodiment, the disclosure relates to methods for formulating nutritionally useful feed additives as co-products of the above-referenced methods for improving nutritional characteristics of a fibrous food product.

The processes for producing fermentation products includes the production of a large number of fermentation products comprising but not limited to alcohols (in particular ethanol) ; acids, such as citric acid, itaconic acid, lactic acid, gluconic acid, lysine ; ketones; amino acids, such as glutamic acid, but also more complex compounds such as antibiotics, such as penicillin, tetracyclin ; enzymes; vitamins, such as riboflavin, B12, beta-carotene; hormones, such as insulin. Preferred is drinkable ethanol as well as industrial and fuel ethanol.

Processes for producing fermentation products, such as ethanol, from a starch or lignocellulose containing material are well known in the art. The preparation of the starch- containing material such as corn for utilization in such fermentation processes typically begins with grinding the corn in a dry-grind or wet-milling process. Wet-milling processes involve fractionating the corn into different components where only the starch fraction enters into the fermentation process. Dry-grind processes involve grinding the corn kernels into meal and mixing the meal with water and enzymes. Generally two different kinds of dry-grind processes are used. The most commonly used process, often referred to as a "conventional process," includes grinding the starch-containing material and then liquefying gelatinized starch at a high temperature using typically a bacterial alpha-amylase, followed by simultaneous saccharification and fermentation (SSF) carried out in the presence of a glucoamylase and a fermentation organism. Another well-known process, often referred to as a "raw starch hydrolysis" process (RSH process), includes grinding the starch-containing material and then simultaneously saccharifying and fermenting granular starch below the initial gelatinization temperature typically in the presence of an acid fungal alpha-amylase and a glucoamylase.

In a process for producing ethanol from corn, following SSF or the RSH process the ethanol is distilled from the whole mash after fermentation. The resulting ethanol-free slurry, usually referred to as whole stillage, is separated into solid and liquid fractions (i.e., wet cake and thin stillage containing about 35 and 7% solids, respectively). The thin stillage is often condensed by evaporation into a thick stillage or syrup and recombined with the wet cake and further dried into distillers' dried grains with solubles distillers' dried grain with solubles (DDGS) for use in animal feed.

In another aspect the present disclosure relates to methods of producing ethanol from starch containing material, said method comprising the steps of:
i) Converting starch-containing material to fermentable sugars
ii) Fermentation of the fermentable sugars with a microorganism to fermented mash
iii) Subjecting the fermented mash after the fermentation process to an enzyme composition according to the present disclosure
iv) Separation of the ethanol in the fermented mash by distillation

Converting starch-containg material to fermentable sugars can be done by (a) liquefying a starch-containing material and (b) saccharifying the liquefied material obtained in step (a).

The liquefaction is preferably carried out in the presence of an alpha-amylase, preferably a bacterial alpha-amylase or acid fungal alpha-amylase. In an embodiment, a pullulanase, isoamylase, and and/or phytase is added during liquefaction.

Preferred organisms for ethanol production are yeasts, such as e. g. Pichia or Saccharomyces. Preferred yeast according to the disclosure is Saccharomyces species, in particular *Saccharomyces cerevisiae* or baker's yeast. The yeast cells may be added in amounts of 10⁵ to 10¹², preferably from 10⁷ to 10¹⁰, especially 5x10⁷ viable yeast count per ml of fermentation broth. During the ethanol producing phase the yeast cell count should preferably be in the range from 10⁷ to 10¹⁰, especially around 2 x 10⁸. Further guidance in respect of using yeast for fermentation can be found in, e. g.,"The alcohol Textbook" (Editors K. Jacques, T. P. Lyons and D. R. Kelsall, Nottingham University Press, United Kingdom 1999).

The microorganism used for the fermentation is added to the mash and the fermentation is ongoing until the desired amount of fermentation product is produced; in a preferred embodiment wherein the fermentation product is ethanol to be recovered this may, e. g. be for 24-96 hours, such as 35-60 hours. The temperature and pH during fermentation is at a temperature and pH suitable for the microorganism in question and with regard to the intended use of the fermentation product, such as, e. g. , in an embodiment wherein the fermenting organism is yeast and the product is ethanol for recovery the preferred temperature is in the range about 26-34 C, e. g. about 32 C, and at a pH e. g. in the range about pH 3-6, e. g. about pH 4-5.

In another embodiment wherein the fermenting organism is yeast, and the fermented mash is to be used as a beer, the temperature of the mash the preferred temperature is around 12-16 C, such around 14 C.

As mentioned above, the fermenting organism is preferably yeast, e.g., a strain of *Saccharomyces cerevisiae* or *Saccharomyces diastaticus.* In an avantegeous embodiment a yeast strain of *Saccharomyces diastaticus* is used (SIHA Amyloferm®, E. Begerow GmbH&Co, Langenlonsheim, Germany) since their exo-amylase activity can split liquid starch and also dextrin, maltose and melibiose.

In the liquefaction step the gelatinized starch (downstream mash) is broken down (hydrolyzed) into maltodextrins (dextrins). To achieve starch hydrolysis a suitable enzyme, preferably an alpha-amylase, is added. Liquefaction may be carried out as a three-step hot slurry process. The slurry is heated to between 60-95°C, preferably 80-85°C, and an alpha-amylase may be added to initiate liquefaction (thinning). Then the slurry may be jet-cooked at a temperature between 95-140°C, preferably 105-125°C, for about 1-15 minutes, preferably for about 3-10 minutes, especially around about 5 minutes. The slurry is cooled to 60-95°C and more alpha-amylase may be added to complete the hydrolysis (secondary liquefaction). The liquefaction process is usually carried out at a pH of 4.0 to 6.5, in particular at a pH of 4.5 to 6.

The saccharification step and the fermentation step may be performed as separate process steps or as a simultaneous saccharification and fermentation (SSF) step. The saccharification is carried out in the presence of a saccharifying enzyme, e. g. a glucoamylase, a beta-amylase or maltogenic amylase. Optionally a phytase and/or a protease is added.

Saccharification may be carried out using conditions well known in the art with a saccharifying enzyme, e.g., beta-amylase, glucoamylase or maltogenic amylase, and optionally a debranching enzyme, such as an isoamylase or a pullulanase. For instance, a full saccharification process may last up to from about 24 to about 72 hours, however, it is common to do a pre-saccharification for typically 40-90 minutes at a temperature between 30-65°C, typically about 60°C, followed by complete saccharification during fermentation in a simultaneous saccharification and fermentation process (SSF process). Saccharification is typically carried out at a temperature from 20-75°C, preferably from 40-70°C, typically around 60°C, and at a pH between 4 and 5, normally at about pH 4.5.

The most widely used process to produce a fermentation product, especially ethanol, is the simultaneous saccharification and fermentation (SSF) process, in which there is no holding stage for the saccharification, meaning that a fermenting organism, such as a yeast, and enzyme(s), including the hemicellulase(s) and/or specific endoglucanase(s), may be added together. SSF is typically carried out at a temperature from 25°C to 40°C, such as from 28°C to 35°C, from 30°C to 34°C, preferably around about 32°C. In an embodiment, fermentation is ongoing for 6 to 120 hours, in particular 24 to 96 hours.

After or during, but preferably after the fermentation, the fermented mash is subjected to an enzyme composition according to the present disclosure. In an embodiment, the enzyme composition comprises a beta-1,3-glucanase, a xylanase and the mutated CBH II described in the present disclosurel. n another embodiment the enzyme composition comprises further a 1,6-beta-glucanase. In a further embodiment, the enzyme composition comprises a beta-1,3-glucanase, a xylanase, thea mutated CBH II and a protease.

In a particular embodiment, the process of the disclosure further comprises, prior to liquefying the starch-containing material the steps of:
- reducing the particle size of the starch-containing material, preferably by milling; and
- forming a slurry comprising the starch-containing material and water.

The aqueous slurry may contain from 10-55 w/w % dry solids (DS), preferably 25-45 w/w % dry solids (DS), more preferably 30-40 w/w % dry solids (DS) of the starch-containing material. The slurry is heated to above the gelatinization temperature and an alpha-amylase, preferably a bacterial and/or acid fungal alpha-amylase, may be added to initiate liquefaction (thinning). The slurry may be jet-cooked to further gelatinize the slurry before being subjected to an alpha-amylase in step (a).

In a preferred embodiment, the starch containing material is milled cereals, preferably barley or corn, and the methods comprise a step of milling the cereals before step (a). In other words, the disclosure also encompasses methods, wherein the starch containing material is obtainable by a process comprising milling of cereals, preferably dry milling, e. g. by hammer or roller mils. Grinding is also understood as milling, as is any process suitable for opening the individual grains and exposing the endosperm for further processing. Two processes of milling are normally used in alcohol production: wet and dry milling. The term "dry milling" denotes milling of the whole grain. In dry milling the whole kernel is milled and used in the remaining part of the process Mash formation. The mash may be provided by forming a slurry comprising the milled starch containing material and brewing water. The brewing water may be heated to a suitable temperature prior to being combined with the milled starch containing material in order to achieve a mash temperature of 45 to 70°C, preferably of 53 to 66°C, more preferably of 55 to 60°C. The mash is typically formed in a tank known as the slurry tank.

Subsequent to fermentation the fermentation product may be separated from the fermentation medium. The slurry may be distilled to extract the desired fermentation product or the desired fermentation product from the fermentation medium by micro or membrane filtration techniques. Alternatively the fermentation product may be recovered by stripping. Methods for recovering fermentation products are well known in the art. Typically, the fermentation product, e.g., ethanol, with a purity of up to, e.g., about 96 vol. % ethanol is obtained.

Following the completion of the fermentation process, the material remaining is considered the whole stillage. As used herein, the term "whole stillage" includes the material that remains at the end of the fermentation process both before and after recovery of the fermentation product, e.g., ethanol. The fermentation product can optionally be recovered by any method known in the art. In one embodiment, the whole stillage is separated or partitioned into a solid and liquid phase by one or more methods for separating the thin stillage from the wet cake. Such methods include, for example, centrifugation and decanting. The fermentation product can be optionally recovered before or after the whole stillage is separated into a solid and liquid phase.

Thus, in one embodiment, the methods of the disclosure further comprise distillation to obtain the fermentation product, e.g., ethanol. The fermentation and the distillation may be carried out simultaneously and/or separately/sequentially; optionally followed by one or more process steps for further refinement of the fermentation product.

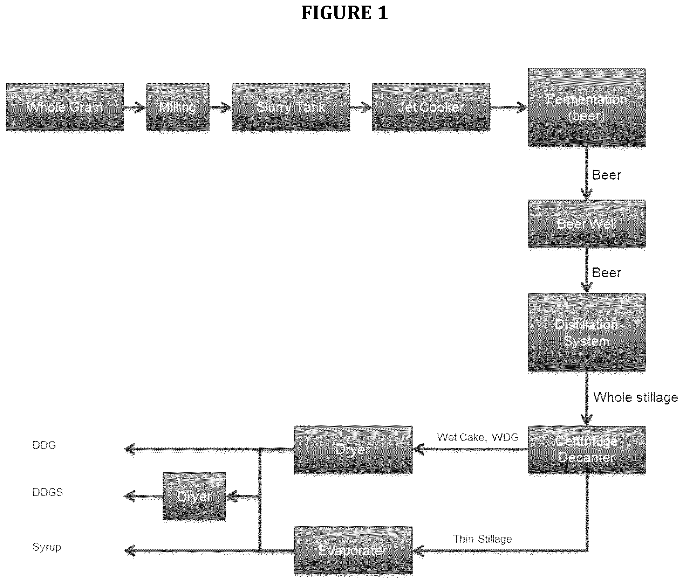

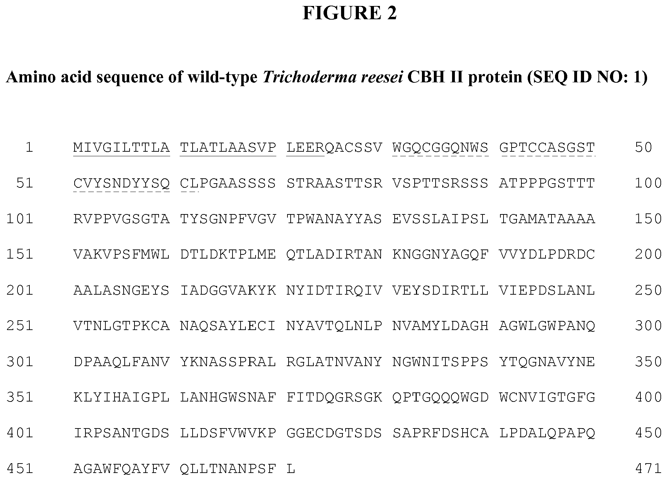

In an embodiment, the aqueous by-product (whole stillage) from the distillation process is separated into two fractions, e.g., by centrifugation: wet grain (solid phase), and thin stillage (supernatant). In another embodiment, the methods of the disclosure further comprise separation of the whole stillage produced by distillation into wet grain and thin stillage; and recycling thin stillage to the starch containing material prior to liquefaction. In one embodiment, the thin stillage is recycled to the milled whole grain slurry. The wet grain fraction may be dried, typically in a drum dryer. The dried product is referred to as distillers dried grains, and can be used as mentioned above as high quality animal feed. The thin stillage fraction may be evaporated providing two fractions (see Fig. 1 and Fig.2), (i) a condensate fraction of 4-6% DS (mainly of starch, proteins, oil and cell wall components), and (ii) a syrup fraction, mainly consisting of limit dextrins and non-fermentable sugars, which may be introduced into a dryer together with the wet grains (from the whole stillage separation step) to provide a product referred to as distillers dried grain with solubles, which also can be used as animal feed. Thin stillage is the term used for the supernatant of the centrifugation of the whole stillage. Typically, the thin stillage contains 4-6% DS (mainly starch and proteins) and has a temperature of about 60-90°C. In another embodiment, the thin stillage is not recycled, but the condensate stream of evaporated thin stillage is recycled to the slurry containing the milled whole grain to be jet cooked. Further details on how to carry out liquefaction, saccharification, fermentation, distillation, and recovering of ethanol are well known to the skilled person.

Methods for dewatering stillage and for extracting oil from a fermentation product are known in the art. These methods include decanting or otherwise separating the whole stillage into wet cake and thin stillage. See, e.g., U.S. Patent Nos. 6,433,146, 7,601,858, and 7,608,729, and U.S. Application Publication No. 2010/0058649. Furthermore, the thin stillage can be evaporated or condensed into syrup or thick stillage from which the oil can be extracted utilizing centrifugation, filtering, heat, high temperature, increased pressure, or a combination of the same. Another way to extract oil is to lower the pH of the thin stillage or syrup. The use of surfactants to break emulsions also enhances oil extraction. Presses can also be used for dewatering. In one embodiment of the disclosure, the presence of beta 1,3-glucanase and/or xylanase in the fermented mash after the fermentation increases the amount of oil in the thin stillage and further the syrup or thick stillage.

The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation as mentioned above. Ethanol with a purity of up to about 96 vol.% can be obtained, which can be used as, for example, fuel ethanol, drinking ethanol, i.e., potable neutral spirits, or industrial ethanol.

A further embodiment pertains to a method of recovering oil from a by-product derived from starch-containing material in a process for producing fermentation products, comprising the steps of:
i) Converting starch containing material to fermentable sugars
ii) Fermentation of the fermentable sugars in a fermentation medium into a fermentation product with a fermenting microorganism
iii) Subjecting the fermented mash after the fermentation and/or the fermentation medium during the fermentation to an enzyme composition according to the present disclosure
iv) Separation of the fermentation product
v) Separation of the by-product
vi) Recovering the oil from the by-product

Methods for dewatering stillage and for recovering oil arising from a fermentation process are known in the art. These methods include decanting or otherwise separating the whole stillage into wet cake and thin stillage. See, e.g., U.S. Pat. Nos. 6,433,146, 7,601,858, and 7,608,729, and U.S. Application Publication No. 2010/0058649. Furthermore, the thin stillage can be evaporated or condensed into syrup or thick stillage from which the oil can be extracted utilizing centrifugation, filtering, heat, high temperature, increased pressure, or a combination of the same. Another way to extract oil is to lower the pH of the thin stillage or syrup. The use of surfactants to break emulsions also enhances oil extraction. Further methods of extracting crude corn oil from corn DDGS are discussed in Sing et. al., "Extraction of Oil From Corn Distillers Dried Grains with Solubles", Transactions of the ASAE 41 (6), 1775-1777 (1998).

After de-oiling of the by-products the carotenoids from the starch-containing materials are mainly comprised in the oil. The oil may be recovered by using solid adsorption materials like bentonite or silica the carotenoid can be easily extracted from the oil, in particular directly in the ethanol production plant and then extracted from the adsorption material for further processing or uses of the adsorption material comprising carotenoids, in particular β-carotene and/or lutein bound to the adsorption material, in particular to bentonite which may directly be used as an animal feed ingredient.

The inventions described and claimed herein are not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

### EXAMPLES

### 1. Identification of CBH2 activity as a key limiting activity during hydrolysis of biomass.

Cellobiohydrolyses play a critical role during depolymerisation of cellulose polymers present in a variety of plant or microbial material. These processes require the complementary action of Cellobiohydrolases, Endoglucanases and Beta-Glucosidases.

To determine the most limiting enzyme activity during hydrolysis of a given biomass (e.g. pretreated wheat straw) the concentrations of individual enzymes were varied in synthetic mixtures. Increased CBH2 concentrations led to increased overall performances of the whole hydrolysis mixtures. Table 1 summarizes these results:

**Table 1**

| **Increase of CBH2 concentration in artificial hydrolysis mixtures** | **Increase of glucose liberation from pretreated wheat straw** |
|---|---|
| 3 x | 27 % |
| 7 x | 46 % |
| 11 x | 73 % |

The reactions were started with 20% pretreated wheat straw and run for 18 h at 45°C. Artificial hydrolysis mixtures contained 1 mg/gram dry substance of the commercial enzyme preparation Econase CE (AB Enzymes) and 20 U/gram dry substance of the Beta-Glucosidase Novo 188. Purified CBH2 preparations were added in the indicated amounts.

### 2. Improvment of the specific activity of the CBH2 variants

Performances of CBHII wildype (of T. reseei) and variant proteins were evaluated during hydrolysis of avicellulose, pretreated wheat straw or barley glucan and variants showing up to 100% improved specific activity were identified. The improvement factors generated from either protein concentration measurement via Bradford or ELISA were very similar and showed a good correlation. The Genotypes of the improved variants were determined as described in example 5. The positions of the identified CBH II amino acid positions were determined from the CBH II wildtype sequence (see figure 2). The following tables summarize the data.

**Table 2: Improvement factors and genotypes of selected CBHII variants during avicellulose hydrolysis**

| **Variant** | **Improvement factor (Bradford)** | **Improvement factor (ELISA)** | **Genotype** |
|---|---|---|---|
| Wildtype | 1,00 | 1,00 | - |
| 1-A | 1,17 | 1,00 | Y53Q, S54V |
| 1-B | 1,21 | 1,10 | A65P, A66Y |
| 1-C | 1,12 | 1,12 | Y53A, S54V |
| 1-D | 1,16 | 1,27 | Q37I, N38K |
| 1-E | 1,43 | 1,24 | H438N |
| 1-F | 1,2 | 1,29 | H438S |
| 2-A | 1,56 | 1,43 | Y53Q, S54V, H438S |
| 2-B | 1,70 | 1,49 | Y53A, S54V, H438N |
| 2-C | 1,56 | 1,56 | Q37I, N38K, A65P, A66Y,H438S |
| 2-D | 1,65 | 1,87 | Q37I, N38K, Y53A, S54V, A65P, A66Y, H438S |

**Table 3: Improvement factors of selected CBHII variants during wheat straw hydrolysis**

| | **Improvement factor** |
|---|---|
| Variant 1-E | 1,25 |
| Variant 2-B | 1,40 |
| Variant 2-D | 2,00 |

### 3. Identification of CBH II variants with improved specific activity on avicellulose

Pure protein samples (one volume) with concentrations of 0.3125, 0.625, 1.25, 2.5, 5.0 or 10.0 µg/ml and two volumes of 5 mU/ml beta-Glucosidase (Megazyme) in 200 mM NaAc pH 5,00 + 0,125% Triton X100 were added to three volumes Avicellulose suspension in distilled water (5% dry substance / 38 µm particles / Fluka). The mixture was incubated at 50°C for at least 2,5 h. After this incubation the glucose content was evaluated by adding one volume of the reaction mixture to six volumes assay containing all components for the enzymatic detection of glucose concentrations (Kit from Megazyme). Following the color development of Resazurin to Resorufin (em535nm/ex595nm) for one hour at 37°C the samples were evaluated by confocal fluorescence spectroscopy. The performance of variants 1-E and 2-D turned out to be significantly improved. As shown in figure 6 variant 1-E proteins showed approximately 35% (at 10 g/ml) improved specific activity compared to the wildtype. At the same protein concentration the specific activity of variant 2-D was approximately 100% improved.

### 4. Identification of CBH II variants with improved specific activity on wheat straw

Pure protein samples (one volume) with concentrations of 1.56, 3.13, 6.25, 12.5, 25, 50 or 100 µg/ml and two volumes of 200 mU/ml beta-Glucosidase (Megazyme) in 200 mM NaAc pH 5,00 + 0,125% Triton X100 were added to three volumes wheat straw suspension in distilled water (2% dry substance). The mixture was incubated at 50°C for at least 2,5 h. After incubation the glucose content was evaluated by adding one volume of the reaction mix to six volumes assay containing all components for the enzymatic detection of glucose concentrations (Kit from Megazyme). Following the color development of Resazurin to Resorufin (em535nm/ex595nm) for one hour at 37°C the samples were evaluated by confocal fluorescence spectroscopy. As shown in Figure 8 variant 1-E proteins showed approximately 23% (at 50 g/ml) improved specific activity compared to the wildtype. At the same protein concentration the specific activity of variant 2-D was approximately 112% improved.

### 5. Identification of CBH II variants with improved specific activity on barley glucan

Pure protein samples (one volume) with concentrations of 0.3125, 0.625, 1.25, 2.5, 5.0 or 10.0 µg/ml and two volumes of 50 mU/ml beta-Glucosidase (Megazyme) in 200 mM NaAc pH 5,00+0,125% Triton X100 were added to three volumes Barley Glucan solution in distilled water (1% w/v; Megazyme). The mixture was incubated at 50°C for at least 2 h. After incubation the glucose content was evaluated by adding one volume of the reaction mixture to six volumes assay containing all components for the enzymatic detection of glucose concentrations (Kit from Megazyme). Following the color development of Resazurin to Resorufin (em535nm/ex595nm) for one hour at 37°C the samples were evaluated by confocal fluorescence spectroscopy. As shown in Figure 9 variant 1-E proteins has improved specific activity compared to the wildtype. At the same protein concentration the specific activity of variant 2-D was also improved.

## Claims

1. An enzyme composition comprising a xylanase, a beta 1,3 glucanase and a polypeptide having cellobiohydrolase II activity comprising an amino acid sequence of CBHII from *Trichoderma reesei,* wherein the amino acid sequence of said polypeptide comprises at least one variation as compared with SEQ ID NO: 1, wherein the variation occurs at position corresponding to amino acid residue 438 of SEQ ID NO: 1, wherein said variation can comprise a substitution, deletion or insertion and wherein the amino acid sequence of said polypeptide comprises an amino acid sequence that is at least 80 percent identical to the CBHII from *Trichoderma reesei* set forth in SEQ ID NO: 1.

2. The enzyme composition according to claim 1, wherein the variation in the polypeptide having cellobiohydrolase II activity is a substitution selected from the group consisting of H438S and H438N.

3. The enzyme composition according to claim 1 or 2, wherein the polypeptide having cellobiohydrolase II activity comprises a further variation at a position selected from the group consisting of: Q37, N38, Y53, S54, A65 and A66.

4. The enzyme composition according to claim 3, wherein the further variation is a substitution selected from the group consisting of Q37I, N38K, Y53A, S54V, A65P and A66Y.

5. The enzyme composition according to any one of claims 1 to 4, wherein the polypeptide having cellobiohydrolase II activity comprises the substitutions selected from the group consisting of:
a) Y53A, S54V, H438N
b) Y53Q, S54V, H438S
c) Q37I, N38K, A65P, A66Y, H438S
d) Q37I, N38K, Y53A, S54V, A65P, A66Y, H438S.

6. The enzyme composition according to any one of claims 1 to 5, wherein the polypeptide having cellobiohydrolase II activity has at least 85, 90, 93 percent, 95 percent, 96 percent, 97 percent, 98 percent or 99 percent identity to SEQ ID NO: 1.

7. The enzyme composition according to any one of claims 1 to 6 comprising further a beta 1,6 glucanase.

8. A method of recovering oil from a by-product derived from starch-containing material in a process for producing fermentation products, comprising the steps of:
i) Converting starch containing material to fermentable sugars
ii) Fermentation of the fermentable sugars in a fermentation medium into a fermentation product with a fermenting microorganism
iii) Subjecting the fermented mash after the fermentation and/or the fermentation medium during the fermentation to an enzyme composition according to any one of claims 1 to 7
iv) Separation of the fermentation product
v) Separation of the by-product
vi) Recovering the oil from the by-product

9. The method according to claim 8, wherein the fermentation product is selected from the group consisting of an acid, an alcohol and hydrogen.

10. The method according to claim 9, wherein the alcohol is selected from the group consisting of ethanol, butanol, propanol, methanol, propanediol and butanediol.

11. The method according to claim 9, wherein the acid is selected from the group consisting of lactic acid, propionic acid, acetic acid, succinic acid, malic acid, butyric acid and formic acid.

12. The method according to any one of claims 8 to 11, wherein the by-product or residue is a fibrous by-product selected from the group consisting of spent brewer's grains, dried distiller's grains, dried distiller's soluble, distiller's dried grains with soluble, wet grains, and mixtures thereof.

13. The method according to any one of claims 8 to 12, wherein the fermented mash is derived from a process of producing a fermentation product utilizing whole grain obtained from cereals as feedstock, the fermentation product is ethanol, the ethanol is separated by distillation and the improved by-product is Dried Distillers Grains with Solubles (DDGS).

14. The method according to any one of claims 8 to 13, wherein the starch containing material is selected from the groups consisting of corn, wheat, barley, rye, millet, sorghum, and milo.

15. The method according to any one of claims 8 to 14, wherein the wherein the microorganism is a bacteria, yeast or fungi.

## Patentansprüche

1. Enzymzusammensetzung umfassend eine Xylanase, eine Beta-1,3-Glucanase und ein Polypeptid mit Cellobiohydrolase-II-Aktivität, umfassend eine Aminosäuresequenz von CBHII aus *Trichoderma reesei,* wobei die Aminosäuresequenz des Polypeptids mindestens eine Variation im Vergleich zu SEQ ID NO: 1 umfasst, wobei die Variation an einer Position auftritt, die dem Aminosäurerest 438 von SEQ ID NO: 1 entspricht, wobei die Variation eine Substitution, Deletion oder Insertion umfassen kann und wobei die Aminosäuresequenz des Polypeptids eine Aminosäuresequenz umfasst, die zu mindestens 80 Prozent identisch mit der in SEQ ID NO: 1 angegebenen CBHII von *Trichoderma reesei* ist.

2. Enzymzusammensetzung nach Anspruch 1, wobei die Variation des Polypeptids mit Cellobiohydrolase II-Aktivität eine Substitution ist, die aus der Gruppe bestehend aus H438S und H438N ausgewählt ist.

3. Enzymzusammensetzung nach Anspruch 1 oder 2, wobei das Polypeptid mit Cellobiohydrolase II-Aktivität eine weitere Variation an einer Position umfasst, die ausgewählt ist aus der Gruppe bestehend aus: Q37, N38, Y53, S54, A65 und A66.

4. Enzymzusammensetzung nach Anspruch 3, wobei die weitere Variation eine Substitution ist, die aus der Gruppe bestehend aus Q37I, N38K, Y53A, S54V, A65P und A66Y ausgewählt ist.

5. Enzymzusammensetzung nach einem der Ansprüche 1 bis 4, wobei das Polypeptid mit Cellobiohydrolase II-Aktivität die Substitutionen umfasst, die ausgewählt sind aus der Gruppe bestehend aus:
a) Y53A, S54V, H438N
b) Y53Q, S54V, H438S
c) Q37I, N38K, A65P, A66Y, H438S
d) Q37I, N38K, Y53A, S54V, A65P, A66Y, H438S.

6. Enzymzusammensetzung nach einem der Ansprüche 1 bis 5, wobei das Polypeptid mit Cellobiohydrolase II-Aktivität mindestens 85, 90, 93%, 95%, 96%, 97%, 98% oder 99% Identität zu SEQ ID NO: 1 aufweist.

7. Enzymzusammensetzung nach einem der Ansprüche 1 bis 6, ferner umfassend eine Beta-1,6-Glucanase.

8. Verfahren zur Gewinnung von Öl aus einem Nebenprodukt, das aus stärkehaltigem Material stammt, in einem Verfahren zur Herstellung von Fermentationsprodukten, umfassend die Schritte:
i) Umwandlung von stärkehaltigem Material in fermentierbaren Zucker
ii) Fermentation des fermentierbaren Zuckers in einem Fermentationsmedium zu einem Fermentationsprodukt mittels eines fermentierenden Mikroorganismus
iii) Aussetzen der fermentierten Maische nach der Fermentation und / oder des Fermentationsmediums während der Fermentation einer Enzymzusammensetzung nach einem der Ansprüche 1 bis 7
iv) Abtrennung des Fermentationsproduktes
v) Abtrennung des Nebenprodukts
vi) Gewinnen des Öls aus dem Nebenprodukt

9. Verfahren nach Anspruch 8, wobei das Fermentationsprodukt ausgewählt ist aus der Gruppe bestehend aus einer Säure, einem Alkohol und Wasserstoff.

10. Verfahren nach Anspruch 9, wobei der Alkohol ausgewählt ist aus der Gruppe bestehend aus Ethanol, Butanol, Propanol, Methanol, Propandiol und Butandiol.

11. Verfahren nach Anspruch 9, wobei die Säure ausgewählt ist aus der Gruppe bestehend aus Milchsäure, Propionsäure, Essigsäure, Bernsteinsäure, Äpfelsäure, Buttersäure und Ameisensäure.

12. Verfahren nach einem der Ansprüche 8 bis 11, wobei das Nebenprodukt oder der Rest ein faseriges Nebenprodukt ausgewählt aus der Gruppe bestehend aus verbrauchten Biertrebern, getrockneten Brennerkörnern, getrockneten Getreideschlempe, Trockenschlempe, feuchte Körner und Mischungen davon, ist.

13. Verfahren nach Anspruch 1, wobei die fermentierte Maische aus einem Verfahren zur Herstellung eines Fermentationsprodukts unter Verwendung von aus Getreide erhaltenem Vollkorn als Einsatzmaterial stammt, das Fermentationsprodukt Ethanol ist, das Ethanol durch Destillation abgetrennt wird und das verbesserte Nebenprodukt Trockenschlempe (DDGS) ist.

14. Verfahren nach einem der Ansprüche 8 bis 13, wobei das stärkehaltige Material ausgewählt ist aus der Gruppe bestehend aus Mais, Weizen, Gerste, Roggen, Hirse, Sorghum und Milo.

15. Verfahren nach einem der Ansprüche 8 bis 14, wobei der Mikroorganismus ein Bakterium, eine Hefe oder ein Pilz ist.

## Revendications

1. Composition d'enzymes comprenant une xylanase, une bêta-1,3-glucanase et un polypeptide ayant une activité cellobiohydrolase II comprenant une séquence d'acides aminés de CBHII de *Trichoderma reesei,* la séquence d'acides aminés dudit polypeptide comprenant au moins une variation par rapport à la SEQ ID NO: 1, la variation se produisant à la position correspondant au résidu d'acide aminé 438 de la SEQ ID NO: 1, ladite variation pouvant comprendre une substitution, une délétion ou une insertion et la séquence d'acides aminés dudit polypeptide comprenant une séquence d'acides aminés qui est au moins identique à 80 % à la CBHII de *Trichoderma reesei* décrite dans la SEQ ID NO: 1.

2. Composition d'enzymes selon la revendication 1, dans laquelle la variation du polypeptide ayant une activité cellobiohydrolase II est une substitution choisie dans le groupe constitué de H438S et de H438N.

3. Composition d'enzymes selon la revendication 1 ou 2, dans laquelle le polypeptide ayant une activité de cellobiohydrolase II comprend une autre variation à une position choisie dans le groupe constitué de : Q37, N38, Y53, S54, A65 et A66.

4. Composition d'enzymes selon la revendication 3, dans laquelle l'autre variation est une substitution choisie dans le groupe constitué de Q37I, de N38K, de Y53A, de S54V, de A65P et de A66Y.

5. Composition d'enzymes selon l'une quelconque des revendications 1 à 4, dans laquelle le polypeptide ayant une activité cellobiohydrolase II comprend les substitutions choisies dans le groupe constitué de :
a) Y53A, S54V, H438N
b) Y53Q, S54V, H438S
c) Q37I, N38K, A65P, A66Y, H438S
d) Q37I, N38K, Y53A, S54V, A65P, A66Y, H438S.

6. Composition d'enzymes selon l'une quelconque des revendications 1 à 5, dans laquelle le polypeptide ayant une activité cellobiohydrolase II a au moins 85 %, 90 %, 93 %, 95 %, 96 %, 97 %, 98 % ou 99 % d'identité avec la SEQ ID NO: 1.

7. Composition d'enzymes selon l'une quelconque des revendications 1 à 6 comprenant en outre une bêta-1,6-glucanase.

8. Procédé de récupération d'huile à partir d'un sous-produit dérivé d'une matière contenant de l'amidon dans un procédé de production de produits de fermentation, comprenant les étapes de :
i) conversion des matières contenant de l'amidon en sucres fermentescibles
ii) fermentation des sucres fermentescibles dans un milieu de fermentation en un produit de fermentation avec un micro-organisme de fermentation
iii) soumission du moût fermenté après la fermentation et/ou le milieu de fermentation pendant la fermentation à une composition d'enzymes selon l'une quelconque des revendications 1 à 7
iv) séparation du produit de fermentation
v) séparation du sous-produit
vi) récupération de l'huile provenant du sous-produit.

9. Procédé selon la revendication 8, dans lequel le produit de fermentation est choisi dans le groupe constitué d'un acide, d'un alcool et de l'hydrogène.

10. Procédé selon la revendication 9, dans lequel l'alcool est choisi dans le groupe constitué de l'éthanol, du butanol, du propanol, du méthanol, du propanediol et du butanediol.

11. Procédé selon la revendication 9, dans lequel l'acide est choisi dans le groupe constitué de l'acide lactique, de l'acide propionique, de l'acide acétique, de l'acide succinique, de l'acide malique, de l'acide butyrique et de l'acide formique.

12. Procédé selon l'une quelconque des revendications 8 à 11, dans lequel le sous-produit ou le résidu est un sous-produit fibreux choisi dans le groupe constitué des grains de brasserie usés, des grains de distillerie séchés, des grains de distillerie séchés solubles, des grains de distillerie séchés avec des grains humides solubles, et de leurs mélanges.

13. Procédé selon l'une quelconque des revendications 8 à 12, dans lequel le moût fermenté est dérivé d'un procédé de production d'un produit de fermentation utilisant des grains entiers obtenus à partir de céréales comme matière première, le produit de fermentation est l'éthanol, l'éthanol est séparé par distillation et le sous-produit amélioré est des grains de distillerie séchés avec des solubles (DDGS).

14. Procédé selon l'une quelconque des revendications 8 à 13, dans lequel la matière contenant de l'amidon est choisie dans les groupes constitués du maïs, du blé, de l'orge, du seigle, du millet, du sorgho et du gros mil.

15. Procédé selon l'une quelconque des revendications 8 à 14, dans lequel ledit micro-organisme est une bactérie, une levure ou un champignon.
